# EUROPEAN PATENT APPLICATION

(11) **EP 1 944 039 A1**
(43) Date of publication of application: **16.07.2008**
(21) Application number: 06775663.5
(22) Date of filing: 14.09.2006
(51) Int. Cl.: A61K 39/205, A61P 35/00

(54) **DRUG FOR TREATING TUMOR AND USE THEREOF IN THE MANUFATURE OF MEDICAMENTS FOR TREATING TUMOR**

(30) Priority: 16.09.2005 CN 200510047220
(71) Applicant: Sun, Jieguang, Dadong District Shenyang Liaoning 110042 (CN); Sun, Xueran, Dadong District Shenyang Liaoning 110042 (CN)
(72) Inventor: Sun, Jieguang, Dadong District Shenyang Liaoning 110042 (CN); Sun, Xueran, Dadong District Shenyang Liaoning 110042 (CN)
(74) Representative: Stuart, Ian Alexander
(86) International application number: PCT/CN2006/002404
(87) International publication number: WO 2007/031030

(57) **Abstract**

The invention relates to medical technical field, which specifically is a medicine for treating tumor and use thereof in preparation of the medicaments for treating tumor by adopting single active component of Rabies Vaccine for Human Use. The invention, having obvious treatment effect and no adverse side-effect, can adjust body function and improve cell and body fluid immunity. An animal experiment has proved its anti-oxidative and anti-aging effect. Meanwhile the invention has ability to prevent and repair cell, enforce body immunity.

## Description

### FIELD OF THE INVENTION

This invention relates to medical technical field, which specifically is a medicine for treating tumor and its application in preparation of medicaments for treating tumor.

### BACKGROUND OF THE INVENTION

Malignant tumor is severely threatening people's health and life. According to the statistic of World Health Organization (WTO), 7 million people die of cancer every year. Since 1996, more than 10 million people have been diagnosed tumor every year all over the world, and the total number of tumor patients has exceeded 40 million. According to the statistic of Department of Health (DH), in China, there are about 1.6 million people diagnosed tumor, and 1.3 million people die of cancer every year. All patients with tumor exceed 2 million. With the increase of incidence of tumor, anti-tumor study has become a hot point in medical field. Scientists predict that finding out super-antigen of tumor and death code of oncogene is crucial to conquer cancer.

Many kinds of chemotherapeutic or assistant anti-tumor medicines have been put into clinical use so far. In the 21th century, each of synthetic medicines, Chinese herbal medicine and biologic agents takes up a field in therapy of cancer. However, most medicines can only relieve the patients' symptom and physical sign but can't cure the disease. In recent years, large amount of manpower and material resource is involved in R & D of anti-tumor medicines all over the world, hoping to breakthrough soon in near future.

Certain cell mass of normal developing or mature cells of body, under long period stimulus of some harmful factors, over-proliferates or abnormally differentiates, and finally forms neoplasm and mass in local area, which is called tumor. It is different from normal tissue and cells, doesn't follow normal metabolic rules, grows without limit and control, and losses normal death, all of which result in abnormal morph, function and metabolism of cells and lead to the destruction of normal structure and function of organs. Malignant tumor can also invade and spread to the peripheral tissue, even transfer to other organs and proliferates geometrically, resulting in great threat to body or life.

Cancer is a common disease that threatens people's life and health, and there's been no effective medicine which can radically cure it so far. At present, main methods used in the treatment of tumor are operation, radiotherapy, chemotherapy, biologic therapy, and others such as endocrine treatment, Chinese traditional therapy, thermotherapy, radiofrequency ablation therapy and so on. Each of above treatments has their respective best indications and disadvantages.

Medicines commonly used in clinical practice are alkylating agents, anti-metabolic medicines, anti-tumor antibiotics, and anti-tumor vegetable medicines, all of which are effective in killing and inhibiting the grow of tumor cell, but their common disadvantages are great damage to normal cells of body, and some toxic side-effects appears in a case of a long time uses. Therefore, extensive use of these kinds of chemical medicines is limited. Biologic or immune modulators, which belong to biologic products, are used in clinical treatment of the disease so as to perform its function of regulating and increas immunity of human body. For example, IFN, IL-II, et al, are partially effective to certain tumors, such as renal carcinoma and melanoma, nevertheless, they are not the first-line medicine because they are expensive and their therapeutic effects are not definite. Recently, Rabies Vaccine for Human Use and Rabies serum immune globulin have been used to prevent and treat rabies or hydrophobia as only effective medicine, and their therapeutic effect is fine. However, reports on study on the medicines of treating human tumors have not been seen yet.

### SUMMARY OF THE INVENTION

The goal of this invention is to provide the anti-tumor medicine having obvious effect in treatment of tumor without toxic side-effect, and the use thereof in preparation of medicaments for treating tumor.

The medicine for treating tumor and use thereof in preparation of medicaments for treating tumor.

Technological program of this invention:

The medicine for treating tumor: adopting single component of Rabies Vaccine for Human Use (liquid or freeze drying) as tumor-treating and tumor-preventing medicine.

The application of Rabies Vaccine for Human Use in preparation of medicaments for treating tumor: adopting single component of Rabies Vaccine for Human Use (maybe liquid or freeze drying) as tumor-treating and tumor-preventing medicine; .

The Rabies Vaccine used here was made with vero, renal cells of hamsler or chick embryo; The virus which was used for making the Rabies Vaccine used here was Pasteur PM, PV, CTN or AG.

Human clinical dosage of intramuscular injection is 2.5 - 10 IU a day, lasting for 20-30 days as a course; Human clinical dosage of intramuscular injection is 2.5 ~10 IU at intervals of 1 to 7 days, lasting for 20-30 days as a course.

Tumor-treating medicine Rabies Vaccine for Human Use can be used simultaneously with biologic product. Biologic product is used in clinical dosage in a case where dosage of the invention is 2.5~10 IU . Biologic product is one of IFN, thymosin Alpha-1, IL-2, TA, immune RNA and cytokine, or their mixture.

This invention is a biologic product, and the study showed that Rabies Vaccine for Human Use, as a tumor-treating medicine, had apparent anti-tumor action, and satisfactory effect thereof could be seen through both animal tumor modal experiment and clinical observation. The details were as follows:

Compared with a control group, the phagocytosis and digestive function of macrophage in group of the invention was enhanced.

The pharmacological and medicine action experiment of the invention showed that the anti-tumor action of the invention could be certified by *in vivo* experiment on animal with tumor and pathological observation of cancer cell. Some related study also investigated the effects of the invention on body immune and anti-oxidation function. A lot of researches provided dependable scientific basis for further development of the invention. The experiment on animal also showed that not only the invention enhanced anti-oxidation, fight against senium and stamina, but also protected and repaired cells so that it boosted up immunity and produced prophylactic action.

### BRIEF DESCRIPTION OF THE DRAWINGS

Fig 1 is a pathological picture of control group in H22 experiment.
Fig 2 is a pathological picture of the killing action of thymopentin on tumor cell in H22 experiment.
Fig 3 is a pathological picture of the killing action of cyclophosphamide on tumor cell in H22 experiment.
Fig 4 is a pathological picture of the killing action of the invention on tumor cell in H22 experiment.
Fig 5 is a pathological picture of control group in S180 experiment.
Fig 6 is a pathological picture of the killing action of thymopentin on tumor cell in S180 experiment.
Fig 7 is a pathological picture of the killing action of cyclophosphamide on tumor cell in S180 experiment.
Fig 8 is a pathological picture of the killing action of the invention on tumor cell in S180 experiment.

### DETAILED DESCRIPTION OF THE INVENTION

Technological program provided by the invention are described below in details.

### Example 1

Human clinical trial: The medicine was Rabies Vaccine for Human Use (cells for kidney of Shrew, freeze-dry agent with water for injection, bought from market).The patients with tumor were intramuscularly injected with Rabies Vaccine for Human Use , 2.5IU per day, for 60 days (a course was 30 days).

Patient Li: pathological diagnosis: squamous cell carcinoma of cervix, stage IIB. After two courses of CAF chemotherapy and local radiotherapy, clinical symptoms were relieved and the Patient recovered acceptably; checking indicated that ratio of CEA was still high. After using the invention, patient's status improved, and there was no abnormality in pathological smear.

### Example 2

Human clinical trial: The medicine was Rabies Vaccine for Human Use (BSD virus PM, Vero cell), liquid agent, bought from market). The patients with tumor were intramuscularly injected with Rabies Vaccine for Human Use, 2.5IU a day, for 30 days as a course.

Patient Ying: pathological diagnosis: squamous cell carcinoma of esophagus, stage IIIA; Clinical symptoms were relieved and food and drink were well after treating by 20 days of local radiotherapy; After three months more radiotherapy, Rabies Vaccine for Human Use (BSD virus PM) was used by intramuscular injection of 2.5 IU a day for 20 days, and the results showed that patient's status improved, T cell subgroup ratio apparently increased, CT indicated that tumor tissue had become smaller, and physiological indexes were normal.

### Example 3

patient Zhao: primary lesion was not clear, with brain metastasis; pathological diagnosis: astrocyte carcinoma; after gamma knife treatment, cerebral edema and coma happened. Family members requested to use Rabies Vaccine for Human Use (PV stock, liquid agent, active unit 2.5IU, bought from market) for treatment. 10 IU was intramuscularly injected every other day for 27 days (a course was 30 days), and then the cerebral edema disappeared with good general condition.

### Example 4

Patient Li: primary lesion was not clear, with multiple metastasis in liver; pathological diagnosis: adenocarcinoma, stage IIB; After 3 courses of CAF chemotherapy and local interventional therapy, clinical symptoms were relieved, and the patient recovered dependably, but CEA was still high when rechecked. Then the patient was treated by the invention (CNT stock, liquid agent, bought from market) and thymosin Alpha-1. Rabies Vaccine for Human Use (active 2.5 IU) was intramuscularly injected 5 IU on the first day, then 2.5 IU every 3 days, for 28 days as a course. Thymopetides (Biologic product): was intramuscularly injected, 1 ramus (1.6mg) once, twice a week, for 28 days as a course according to clinical program. After intermittent treatment, patient's condition improved, CT indicated that tumor had become smaller.

### Example 5

Patient Zhou: pathological diagnosis: squamous cell carcinoma of cervix, stage IIC, with chemotherapy of CAF for one course after an operation, the clinical symptoms were relieved and the patient recovered acceptably. checking, indicated that CEA was still high. Rabies Vaccine for Human Use (BSD virusPM liquid active unit 2.5 IU) of the invention was associated with IFN (biologic product) for treatment. The patient was intramuscularly injected with Rabies Vaccine for Human Use,7.5 IU every 7 days, for 24 days as a course, and at the same time IFN, a ramus (1 million u) a time, once every other day, for 24 days as a course according to clinical therapy program. After combined treatment, the patient's condition improved, and there was no abnormality on pathological smear.

### Example 6

Patient Ma: pathological diagnosis: nasopharyngeal carcinoma, stage IIB; After 2 courses of CAF chemotherapy and local radiotherapy, the clinical symptoms were relieved, and the patient recovered acceptably; checking indicated CEA was still high. Rabies Vaccine for Human Use ( dok liquid) was associated with biologic product(IL-2 and TF) for treatment. Rabies Vaccine for Human Use (active unit 2.5 IU) was intramuscularly injected 10 IU every 4 days, for 25 days as a course and IL-2, a ramus (1 million u) once, once every other day, for 25 days as a course; TF was orally administrated, 1 bottle (10 ml) a time, once a day, according to clinical therapy program. After combined treatment, the patient's condition improved, and CT film indicated the tumor lesion had become smaller.

### Example 7

Patient Xu: pathological diagnosis: adenocarcinoma of superior fissure of the lung; After CAF chemotherapy for 3 courses and local radiotherapy, the clinical symptoms were relieved, and the Patient recovered acceptably. Rabies Vaccine for Human Use (AG, liquid) was associated with biologic product(immune RNA and cytokine) for treatment. Rabies Vaccine for Human Use (active unit 2.5 IU), was intramuscularly injected, 2.5 IU a time, once a day, for 20 days as a course. ; Immune RNA , a ramus a time, once a day, for 20 days as a course according to clinical therapy program; Cytokine, a ramus(1 ml) a time, once every other day, for 20 days as a course. After combined treatment, the patient's condition improved, and CT film indicated the tumor had become smaller.

### Example 8, healthy people

400 healthy persons (over 45 years old, 252 are mail and 148 are female) are divided by random into two groups, an experimental group and the control group. In the experimental group, according to immune programme, the invention was intramuscularly injected intermittently, 2.5 IU a time. A detection test showed that antibodies could be rapidly produced by body. After 2 years follow-up, there's no tumor or malignant disease developed. Case of influenza was rare, too. In the control group, 3 persons had cancer, one lung cancer, one oesophagus cancer and one nasopharynx cancer. Therefore the medicine of the invention can prevent cancer occurrence.

### Example 9 (to verify the inhibiting action of the invention on tumor by using mice with tumor)

The purpose is to investigate the inhibiting action of the invention on mice with H22 hepatic carcinoma and S180 solid tumor, and the action on prolongating the life of mice with Ehrlich's ascites carcinoma.

Medicine being investigated: Rabies Vaccine for Human Use of the invention, was bought from market; . 0.1 U/ml of its Suspension liquid was prepared by adding normal saline.

### Experimental materials:

1. Animal: mice of Kunming species, weighted 20±2g/each mouse, 20 in each group, which were provided by experimental animal department of China Medical University. Certificated number of experimental animal is Liaoshidongzi No. 521.
2. S180 sarcoma, H22 hepatic carcinoma and Ehrlich's ascites carcinoma were provided by medicine institute of Beijing Chinese Academy of Medical Sciences.
3. The feedstuff of mice, which was provided by Shenyang experimental animal granular feedstuff factory, was granular.
4. Contrast medicines
   1) Cyclophosphamide( for chemotherapy; Batch No.05060721) was an injection, which was provided by Jiangsu Heng Medicine CO. Ltd.. And 3mg/ml of its solution was prepared by adding normal saline.
   2) Hypersonsortium (biological drug, Batch No.20050703), was provided by Shenyang Xiehe Biological Tragacanth CO. Ltd.. 1mg/ml of its solution was prepared by adding normal saline.
   3) Thymosin Alpha-1 injection(biological drug, registration No.H20050339 Batch No.P005D), was provided by American Saisheng Medicine (HongKong) International Limited Company.
5. Control group: normal saline

Method: experiments were carried out according to "New Medicine Pre-Clinical Anti-Tumor Medicine Instruction Principle" and "the Third Nationwide Tumor Pharmacology and Chemotherapy Conference Formulate Program". Tumor fluid was withdrawn from mice with tumor which were growing well under asepsis condition, and it was attenuated to 10³ cells / ml cell suspension with normal saline.

### 1. Tumor-inhibiting experiment

### 1.1 Solid tumor inhibiting experiment

0.2ml diluted tumor fluid was subcutaneously injected in right axillary into the mice that was consisted of half of the male mice and half of the female mice. Next day, the mice were weighed and randomly divided into groups. The medicine of the invention was injected into tumor tissue of the mice, from the fourth day after subcutaneous injection, once a day for successive 5 days (control group: injection of 0.2ml normal saline into tumor tissue). The animals were killed on the 15^{th} day after medicine withdrawal, and their subcutaneous tumor masses were isolated and weighed. Pathological examinations and calculation of the mean weight and inhibition rate of the tumor were carried out.

### 1.2. Experiment of prolongation rate of life

0.2ml diluted tumor fluid was injected into abdominal cavity of the mice that was consisted of half of the male mice and half of the female mice. Next day, the mice were weighed and randomly divided into groups. The medicine of the invention was injected into abdominal cavity of the mice from the fourth day after subcutaneous injection, once a day for successive 15 days (control group: injection of 0.2ml normal saline into abdominal cavity). The mice were observed after medicine withdrawal until the mice were dead. The time of death and the weight of each mouse were recorded.

### Result of the experiment

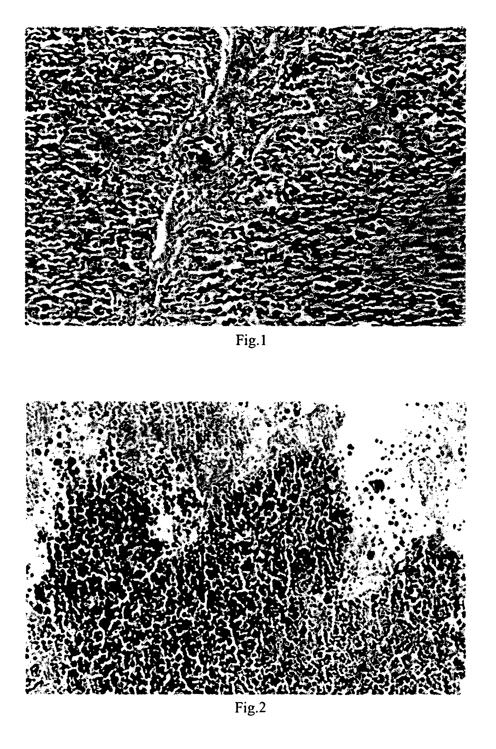

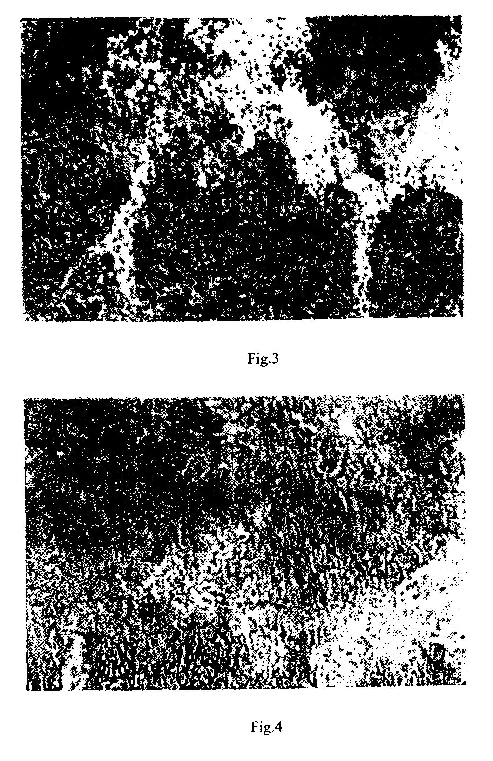

In H22 experiment, anatomic gross appearance, the capsule of the tumor was relatively complete, except for a little of corruption appearance. And in the capsule there was a lot of liquid (mainly bloody). Corruption of most tumor tissue appeared, and pathologic plates showed that many necroses in tumor tissue appeared, nucleuses of the tumor tissue were stained light, many structures in cells disappeared or degenerated, and the tumor tissue became smaller. The necrosis degree in the group of the vaccine of the invention was larger, compared with cyclophosphamide and thymopentin. Refers to Fig 1, the pathological picture of control group of H22 experiment, visual field was full of tumor cells, the nucleuses were stained deeply, and cytoplasm was less; . Refers to Fig 2, the pathological picture of thymopentin group of H22, it showed a little of tumor cell necrosis and degeneration, and large numbers of tumor cells in local area;. Fig 3, the pathological picture of cyclophosphamide group of H22 experiment, tumor tissue in which necrotic and degenerated tissue scattered, dispersed partially; . Refers to Fig 4, the pathological picture of the vaccine group of H22 experiment, large amount of tumor tissue was necrotic and degenerative, liquated locally, and nucleuses were stained light, tumor tissue became less in the center.

In S180 experiment, anatomic gross appearance, the capsule of the tumor was relatively complete, except for a little of corruption and denaturation And in the capsule there was a lot of liquid which was semi-translucent and light yellow, and of which only a small amount was semi-bloody.

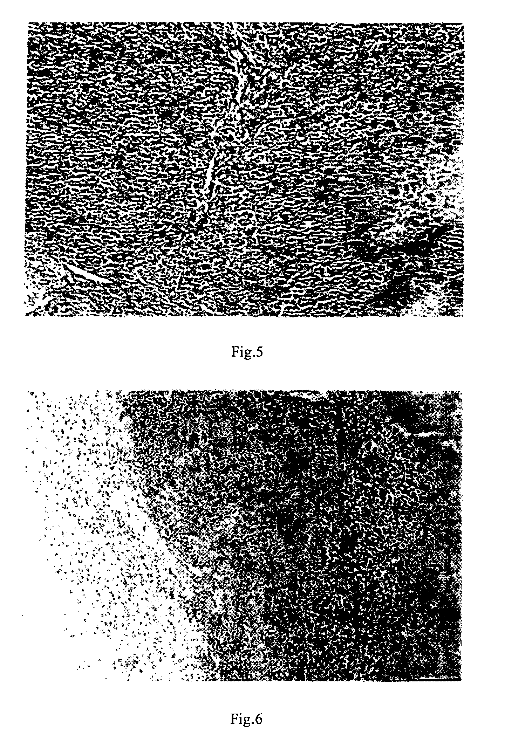

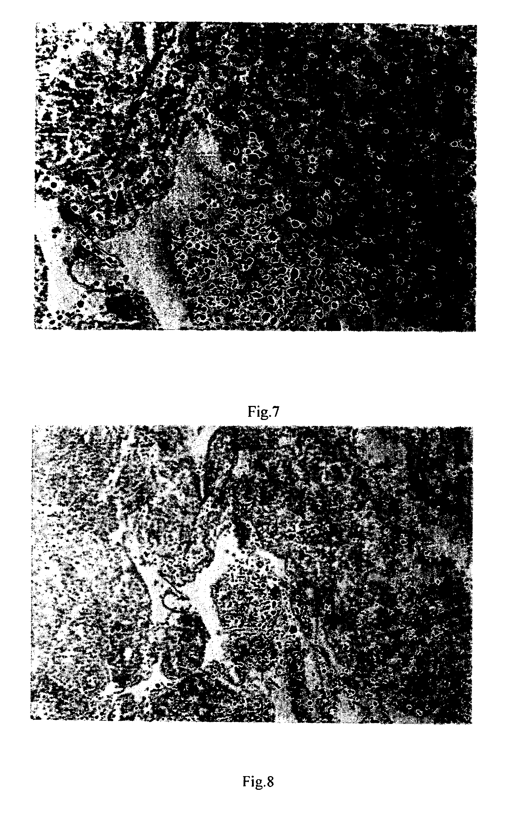

Seen from pathological picture: in the group of the vaccine of the invention of S180 experiment, there was large area of tumor tissue stained light, the membrane of nucleuses disappeared, the nucleuses were stained light, and the structure of the tissue degenerated or disappeared(Fig 5, the pathological picture of S180 in control group, visual field was full of tumor cells, the nucleuses were stained deeply, and the cytoplasm was little; Fig 6, the pathological picture of S180 in thymopentin group, a few tumor cells were necrotic and degenerative, and large amount of tumor tissue could be seen in local area; Fig 7, the pathological picture of S180 in cyclophosphamide group, tumor tissue in which necrotic and degenerated tissue scattered, dispersed partially; Fig 8, the pathological picture of S180 in the group of the vaccine of the invention, large amount of tumor tissue was necrotic and degenerative, liquated locally, and nucleuses were stained light, tumor tissue became less in the center).

In the Ehrlich's ascites carcinoma experiment, there was apparent prolongation of life in the group of the vaccine of the invention, compared with the control group or other groups.

Conclusion of the experiments: the medicine of the invention had the action of inhibition on H22 and S180. Pathological picture showed strong destructive action on tumor cells. The medicine obviously prolonged life of animals with tumor.

### Conclusion of the experiments

Inhibiting rate, degree of ascites carcinoma and life prolongation rate, therapeutic effects of the medicine were certified through injection of the medicine into mice with tumor for the successive 5, or 15 days and then the observation of tumor weight.
1)Evaluation of therapeutic effect on solid tumor: Inhibiting rate was about 40%, the highest was 41.8%. Successive 3 experiments showed stable therapeutic effects and statistic significant difference (P<0.05). The medicine had good effects on solid tumor.
2)Evaluation of therapeutic effects on ascites carcinoma: Compared with other groups, life prolongation rate in the vaccine group of the invention was more than 50%. Successive 3 experiments showed stable therapeutic effects and statistic significant difference (P<0.05) .The medicine had good effects on ascites carcinoma.

Experiment result: the therapeutic effects on both solid tumor and ascites cancer were good and stable, with strong tumor-inhibiting action. There was no obvious toxic side-effect during the experiments.

### 2. Action on regulating immune function, increasing deintoxication, regulating repair of injury of chemotherapy or radiotherapy and preventing the injury

Chemotherapeutic agents are a kind of cytotoxin, which can inhibit immune function. Some animal experiments of immune and anti-oxidation suggested that the medicine of the invention had antibody level increase, enhanced chemotaxis and adhesion of phagocytes, and promoted the release of intracellular active substances which improved phagocytosis and digestive function which were inhibited by chemotherapy.

This invention investigated the function of the medicine of the invention to regulate repair of injury of radiotherapy on mice with tumor. Changes of inhibition rate, anti-radiation, anti-oxidation, anti-fatigue and immune function improvement were observed mainly.

### Animal experiments certified that:

1) After the medicine of the invention was applied, the function of mononuclear phagocytes was apparently improved, and the phagocytosis index and activity were 0.3480 and 8.2870 respectively. Data were processed and there was significant difference between the vaccine and control group, as shown in Table 1.

**Table 1 Experimental results of the function of mononuclear phagocytes**

| Group | No. of animal | Type of administration | Dosage (mg/kg) | Index of phagocytosis,K | | Phagocytosis activity,α | |
|---|---|---|---|---|---|---|---|
| Control | 10 | Peritoneal | 0.2 mL N.S. / each | 0.0867 | ± | 5.1542 | ± |
| | | injection | | 0.0058 | | 0.3016 | |
| Chemotherapy | 10 | Peritoneal | 20mg/kg / each | 0.0744 | ± | 4.4614 | ± |
| | | injection | | 0.0081 | | 0.2413 | |
| Hyperconsortium | 10 | Peritoneal | 100mg/kg / each | 0.3331 | ± | 7.8440 | ± |
| | | injection | | 0.0177 | | 0.5096 | |
| Hyperconsortium+chemothetapy | 10 | Peritoneal | 100mg+20 mg /kg / each | 0.2868 | ± | 7.0877 | ± |
| | | injection | | 0.0348 | | 0.4307 | |
| Vaccin | 10 | Peritoneal | 0.625U/kg / each | 0.3480 | ± | 8.2870 | ± |
| | | injection | | 0.0120 | | 0.3221 | |
| Vaccin+chemotherapy | 10 | Peritoneal | 0.625U+20mg/kg / each | 0.2969 | ± | 7.2141 | ± |
| | | injection | | 0.0115 | | 0.3674 | |
| Thymosin Alpha-1 | 10 | Peritoneal | 0.3mg/kg / each | 0.3473 | ± | 7.9583 | ± |
| | | injection | | 0.0130 | | 0.5069 | |
| Thymosin Alpha-1s +chemotherapy | 10 | Peritoneal | 0.3mg+20 mg /kg / each | 0.2923 | ± | 7.8088 | ± |
| | | injection | | 0.0041 | | 0.3159 | |

| | | | | | | | |
|---|---|---|---|---|---|---|---|
| Note: comparison between group, P<0.1. | | | | | | | |

2) After administration of the medicine of the invention, the phagocytosis rate of macrophage in the abdominal cavity of mice reached up to 35.02%, and the index of phagocytosis also increased greatly, reaching up to 1.02. Besides, the digestive function of macrophage increased as well, resulting in the increase of serum HD50 and strong regulation function to antibody formation after chemotherapy (regulating function of immune function).
3) Lower peripheral WBC level, which had been reduced by chemotherapy of large dosage of cyclophosphamide and ordinarily needed a long time to recover, could return to normal level rapidly. The medicine of the invention could return to normal within 7-10 days (detoxication).
4) Tumor-inhibiting rate of mice with tumor was 41.8% with exclusive administration of the medicine of the invention. And the rate reaches up to 59.53% with both administration of the medicine of the invention and chemotherapy (synergism).
5) The medicine of the invention could enhance activity of SOD in the liver of mice. SOD level was 662.97ug/ml with administration of the medicine of the invention, and SOD level was 452.69ug/ml with both administration of the medicine of the invention and chemotherapy. SOD level in the group with the medicine of the invention was higher than that in the control group (P>0. 05) (anti-oxidation action).
6) The medicine of the invention had apparent injury repair of radiation. The medicine of the invention prolonged lifetime of the animal exposed to radiation. And the head skin and mucosa were mildly injured. But in the control group, the animal were severely injured, ulcer of head skin and mucosa were severe with depilation of large area and diarrhea. There was no apparent rise in micronucleus rate of bone marrow of the mice in the vaccine group of the invention, and 30 days survival rate was 82.07% and mean month survival time was 24.62 days, both of which were higher or longer than the control group (regulation function of injury repair of radiotherapy and chemotherapy).
7) The medicine of the invention prolonged swimming time of healthy mice 75% longer, so it obviously enhanced stamina (prevention action).

**Table 2 Experimental result of the function of swimming time of mice**

| Group | No. of animal | Type of administration | Dosage (mg/kg) | Swimming time (min) | Elongation index (%) |
|---|---|---|---|---|---|
| Control | 10 | Peritoneal | 0.2 mL N.S. / each | 13.8±3.85 | |
| | | injection | | | |
| Hyperconsortium | 10 | Peritoneal | 100mg/kg/ each | 14. 0±3.62 | 14.4% |
| | | injection | | | |
| Vaccin | 10 | Peritoneal | 0.625U/kg/each | 24.2±4.21 | 75.1% |
| | | injection | | | |
| Thymosin Alpha-1 | 10 | Peritoneal | 0. 3mg/kg/ each | 18. 9±2.98 | 37.5% |
| | | injection | | | |

| | | | | | |
|---|---|---|---|---|---|
| Note: comparison between group, P<0.1. | | | | | |

The study showed that the medicine of invention had many biological mediation actions, and especially more effective action on tumors induced by infection of human papilloma virus, that is, it was able to improve side-effects of treatment of malignant tumor, and it had immunoenhancement and corresponding prevention action on healthy persons. Its application prospect is broad owing to huge market of tumor treatment and rehabilitation.

## Claims

1. A medicine for treating tumor, **characterized in that** adopting active component of Rabies Vaccine for Human Use.

2. The medicine for treating tumor according to requirment1, **characterized in that** the said Rabies Vaccine for Human Use is liquid or frozen dry powder.

3. A use of the medicine in preparation of medicaments for treating tumor according to the claim 1, **characterized in that** the active component is Rabies Vaccine for Human Use as a medicine for treating and preventing cancer or tumor.

4. The use of the medicine in preparation of medicaments for treating tumor according to the claim 3, **characterized in that** the said Rabies Vaccine for Human Use is made from Vero, kidney cell of shrewmouse or chicken embryo.

5. The use of the medicine in preparation of medicaments for treating tumor according to the claim 3, **characterized in that** the said Rabies Vaccine for Human Use is made from rabies virus pasteurium including PM, PV, CTN or AG.

6. The use of the medicine in preparation of medicaments for treating tumor according to the claim 3, **characterized in that** the said Rabies Vaccine for Human Use is liquid or frozen dry powder.

7. The use of the medicine in preparation of medicaments for treating tumor according to the claim 3, **characterized in that** clinical dosage of the said Rabies Vaccine for Human Use is 2.5 IU -10 IU a day, for 20~30 days as a treatment cycle.

8. The use of the medicine in preparation of medicaments for treating tumor according to the claim 3, **characterized in that** clinical dosage of the said Rabies Vaccine for Human Use is 2.5 IU -10 IU a time, once at intervals of 1 to 7days , for 20~30 days as a treatment cycle.

9. The use of the medicine in preparation of medicaments for treating tumor according to the claim 3, **characterized in that** the said Rabies Vaccine for Human Use matching biology preparation as a medicine for treating cancer, the said Rabies Vaccine for Human Use is 2.5IU~10 IU, biology products is clinical dosage.

10. The use of the medicine in preparation of medicaments for treating tumor according to the claim 9, **characterized in that** the said biological product is one of IFN, Thymosin Alpha-1, IL-2, TA, immune RNA and cytokine, or their mixture by using clinical dosage.
